# EUROPÄISCHE PATENTSCHRIFT

(11) **EP 0 495 769 B1**
(45) Veröffentlichungstag und Bekanntmachung des Hinweises auf die Patenterteilung: **13.09.1995**
(21) Anmeldenummer: 92890009.1
(22) Anmeldetag: 15.01.1992
(51) Int. Cl.: C12M 1/40, C12M 3/02

(54) **Reaktor zur Durchführung biologischer Reaktionen mittels Biokatalysatoren**
Reactor for performing biological reactions using biocatalysts
Réacteur pour la mise en oeuvre des réactions biologiques utilisant biocatalyseurs

(30) Priorität: 16.01.1991 AT 93/91
(43) Veröffentlichungstag der Anmeldung: 22.07.1992
(73) Patentinhaber: VOGELBUSCH GESELLSCHAFT m.b.H., A-1050 Wien (AT)
(72) Erfinder: Katinger, Hermann W.D., Dipl.-Ing. Dr., A-1190 Wien (AT); Reiter, Manfred, Dipl.-Ing. Dr., A-1160 Wien (AT); Blüml, Gerald, Dipl.-Ing., A-1160 Wien (AT); Zach, Nicolaus, A-1080 Wien (AT); Gaida, Theodor, A-1020 Wien (AT)
(74) Vertreter: Itze, Peter, Dipl.-Ing.

(56) Entgegenhaltungen:
- EP-A- 0 095 804
- WO-A-86/05202
- WO-A-90/11345
- CH-A- 525 959

## Beschreibung

Die Erfindung bezieht sich auf einen Reaktor zur Durchführung biologischer Reaktionen mittels Biokatalysatoren mit einem Basisteil, in welchem eine Rühreinrichtung und Überwachungs- und Steuerorgane bzw. -geräte angeordnet sind, und einem dicht aufsetzbaren Deckel.

Bei diesen bekannten Ausbildungen handelt es sich um herkömmliche Reaktoren für den chargenweisen oder kontinuierlichen Betrieb, wobei bei diesen Systemen die Biokatalysatoren mit dem Substrat ausgetragen werden und dann nachträglich aus dem Substrat separiert werden müssen.

Es sind auch bereits Reaktoren bekannt, bei welchen die Biokatalysatoren auf einer Trägermatrix, z.B. sogenannten "Microcarrier", immobilisiert sind, bzw. bilden die Biokatalysatoren selbst durch Zusammenballungen eine eigene Matrix. Bei diesen Reaktoren sind die Microcarrier bzw. die Biokatalysatorenmatrix in einem Zylinder angeordnet, welcher von unten her mit Substrat durchströmt wird, und zwar in solchem Maße, daß die Microcarrier bzw. die Biokatalysatorenmatrix in Form eines Fließbettes fluidisiert sind. Diese Matrix bzw. die Microcarrier werden von Substrat durchströmt, bleiben jedoch aufgrund ihres spezifischen Gewichtes und der darauf abgestimmten Strömungsgeschwindigkeit in dem Zylinder zurück, wogegen das umgewandelte Substrat am oberen Ende abgezogen und im Kreislauf geführt wird. Die Umwälzleitung wird einem Gasaustauscher zugeführt, in welchem das Substrat mit Sauerstoff angereichert und das gebildete Kohlendioxid abgezogen wird. Weiters sind in der Umwälzleitung dann noch Heizorgane, pH-Meßorgane, Sauerstoffsonden, Temperatursonden und eine Umwälzpumpe angeordnet. Derartige Ausbildungen haben den Nachteil, daß damit nur so viel Sauerstoff in das Substrat eingebracht werden kann, als darin in Lösung geht, so daß innerhalb des Fließbettes ein Abfall der Sauerstoffkonzentration so stark vor sich geht, daß im oberen Bereich des Fließbettes der Sauerstoffanteil des Substrates so niedrig ist, daß die weitere Reaktion behindert wird.

Der Erfindung liegt die Aufgabe zugrunde, einen Reaktor der eingangs genannten Art zu schaffen, mit welchem den Biokatalysatoren ein über die gesamte Länge ausreichendes Angebot an dem zur Reaktion benötigten Gas zur Verfügung steht.

Erfindungsgemäß wird diese Aufgabe dadurch gelöst, daß zwischen dem Basisteil und dem Deckel ein Zwischenteil eingesetzt ist, der bei Verwendung als Fließbettreaktor und / oder Festbettreaktor in seinem unteren Bereich einen gasblasendurchlässigen Träger, insbesondere eine Trägerplatte, für eine mit Biokatalysatoren beladene Matrix bzw. Biokatalysatormatrix aufweist, wobei eine vom oberen Bereich des Zwischenteiles ausgehende, zum Basisteil führende Umwälzleitung vorgesehen ist, und wobei die Rühreinrichtung als Umwälzpumpe ausgebildet ist. Dadurch wird erreicht, daß eingebrachte Gasblasen die Trägerplatte durchsetzen können und damit das Gas in blasenförmigem Zustand in den Fließbett- bzw. Festbetteil einbringen können, wobei die Gasblasen durch das Durchtreten der Trägermatrix bzw. der Biokatalysatormatrix immer wieder eine neue Grenzfläche zwischen dem Blaseninhalt und dem Substrat schaffen, wodurch eine völlige Ausnützung des bläschenförmig eingebrachten Gases erreicht wird. Außerdem wird durch den Austausch der Grenzflächen erreicht, daß ein Gasaustausch dahingehend erfolgen kann, daß Sauerstoff aus den Blasen entweicht und dafür als Stoffwechselprodukt Kohlendioxid aus dem System ausgebracht wird. Weiters wird durch die erfindungsgemäße Ausbildung ein herkömmlicher Reaktor in einen Fließbettreaktor bzw. Festbettreaktor umgewandelt, wodurch die für einen herkömmlichen Reaktor zur Verfügung stehenden Überwachungs- und Steuerorgane bzw. -geräte auch in den Fließbettreaktor einsetzbar sind.

Vorteilhafterweise kann der Zwischenteil modular austauschbar sein, so daß die teuren Geräte des Basisteiles für eine Vielzahl von Reaktorarten anwendbar sind. Weiters kann der durch die Trägerplatte gebildete Träger eine hydrophobe Oberfläche und eine Durchlässigkeit von 3 bis 12 %, vorzugsweise 5 bis 7,5 %, aufweisen. Dadurch wird erreicht, daß Luftbläschen ungehindert durch die Trägerplatte hindurchtreten können, ohne an der Unterseite große Blasen zu bilden, die dann eruptionsartig durch die Trägerplatte hindurchtreten und die mit Biokatalysatoren beladene Matrix bzw. die Biokatalysatorenmatrix nach oben mitreißen. Für eine besonders gute Gaseinbringung kann der gasblasendurchlässige Träger als statischer Mischer ausgebildet sein, wodurch eine nochmalige intensive Durchmischung der Reaktorflüssigkeit mit dem Gas erzielt wird.

Als besonders einfache Ausbildung kann die Umwälzleitung als zentrales, gegebenenfalls durch ein Gitter abgeschlossenes Rohr, ausgebildet sein, das die Trägerplatte durchsetzt und zur Rühreinrichtung führt. Damit wird Energieverlusten durch ein außenliegendes Rohr vorgebeugt, wobei abgesehen vom geringeren Platzbedarf die erfindungsgemäße Vorrichtung besonders leicht zu sterilisieren und zu reinigen ist. Zur Erhöhung der Pumpwirkung der Rühreinrichtung kann das die Umwälzleitung bildende zentrale Rohr an seinem unteren Bereich mit einem Kragen versehen sein, der außen das die Pumpe bildende Flügelrad der Rühreinrichtung übergreift, wobei die Rühreinrichtung als Axialförderer ausgebildet ist. Dadurch wird eine besonders gute Umwälzung erzielt, wobei durch das zentrale Abziehen der Flüssigkeit eine gleichmäßige Umwälzung erreicht wird. Dabei kann zwischen der Umwälzleitung und dem Kragen ein sich konisch zum Kragen hin erweiternder Übergangsbereich vorgesehen sein, was eine strömungstechnisch besonders bevorzugte Ausbildung ergibt. Um eine in beiden Richtungen gleich wirksame Pumpeffizienz zu erzielen, kann der Flügel der Rühreinrichtung etwa in der Längsmitte des Kragens angeordnet sein. In dem Zwischenraum zwischen Kragen und Reaktorinnenwandung können Schikanen zur Verhinderung des Rotierens der Reaktorflüssigkeit eingesetzt sein, wodurch die Flüssigkeit in im wesentlichen vertikaler Richtung von unten auf die Trägerplatte auftrifft und diese daher gleichmäßig von unten nach oben durchströmt, so daß gleichmäßige Strömungsverhältnisse im Fließbett- bzw. Festbett erreicht sind. Um während des Umwälzens auch Reinigungs- und ähnliche Prozesse ausführen zu können, kann die Wandung der Umwälzleitung wenigstens in Teilbereichen derselben als Filtermembran, z.B.Ultrafiltermembran, oder als Permeationsmembran ausgebildet sein.

Für eine besonders wirkungsvolle Gaseinbringung können in der Umwälzleitung oberhalb der Rühreinrichtung, insbesondere im erweiterten Übergangsbereich, Gaseinbringorgane vorgesehen sein. Dadurch wird das eingebrachte Gas durch die Rühreinrichtung sogleich in der Flüssigkeit fein verteilt und dann als fein verteilte Blasen durch die Trägerplatte in das Festbett bzw. Fließbett eingebracht.

Vorteilhafterweise kann das Flügelrad der Rühreinrichtung scherkraftarm ausgebildet sein, so daß auch Zellen in Suspension überleben und wieder in die Matrix rückführbar sind, ohne daß Lyseprodukte auftreten können, die z.B. auf die mechanische Schädigung der Zellen durch die Rühreinrichtung zurückzuführen sind.

Schließlich kann die Rühreinrichtung drehrichtungsumkehrbar antreibbar sein, wodurch ein festes Packen des Fließbettes oder des Festbettes erreicht wird, bzw. kann auf diese Weise durch Rückspülung eine gute Beimpfung des Fließbettes bzw. Festbettes erreicht werden.

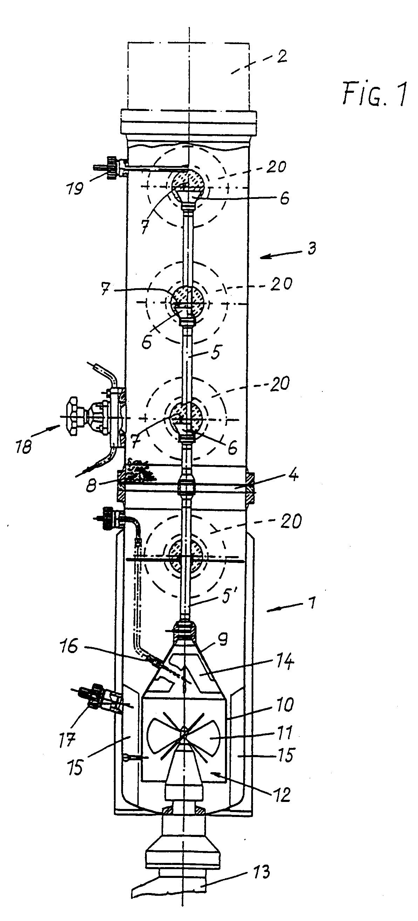

In der
Fig. 1 ist ein Ausführungsbeispiel eines erfindungsgemäßen Reaktors im Vertikalschnitt dargestellt. Die
Fig. 2 - 4 zeigen Kurven betreffend die über den Versuchsablauf erreichten Zelldichten.

Der Reaktor besteht aus einem Basisteil 1, einem Deckel 2 und einem dazwischen angeordneten Zwischenteil 3. Der Zwischenteil 3 ist modular austauschbar, wobei in vorliegendem Fall der Zwischenteil für einen Fließbettreaktor bzw. Festbettreaktor ausgebildet ist. Dazu weist der Zwischenteil 3 in seinem unteren Bereich eine poröse Trägerplatte 4 auf, welche aus hydrophobem Material gebildet ist und eine Durchlässigkeit von 3 bis 12 %, in vorliegendem Fall von 5 bis 7,5 %, aufweist. Die Trägerplatte ist aufgrund ihrer Ausbildung als statischer Mischer wirksam und aufgrund der hydrophoben Oberfläche und der angegebenen Durchlässigkeit gasblasendurchlässig. In dem Zwischenteil 3 ist eine zentrale Umwälzleitung 5 vorgesehen, welche an ihrem oberen Ende mit einem Einlaßtrichter 6 versehen ist. In dem vorliegenden Beispiel sind die Einlaßtrichter 6 an drei verschiedenen Höhen angedeutet, wobei diese Einlaßtrichter je nach Füllhöhe des Reaktors angebracht werden. Die Einlaßtrichter sind an ihrer Oberseite durch ein Gitter 7 abgedeckt, um einerseits die Bildung eines Vortex beim Einsaugen zu verhindern und anderseits auch das Füllmaterial des Festbettes bzw. Fließbettes an einem Einsaugen in die Umwälzleitung zu hindern. Das Füllmaterial ist bei 8 angedeutet.

Das Füllmaterial 8 kann entweder eine Trägermatrix für Biokatalysatoren sein, sogenannte "Microcarrier", welche ein spezielles spezifischen Gewicht aufweisen und eine entsprechend poröse Struktur besitzen, so daß die Biokatalysatoren, z.B. Mikroorganismen, Zellen von Zellkulturen, Enzymketten u.dgl., an dem Microcarrier anhaften können, bzw. sind die Microcarrier von den Organismen durchwachsen. Es kann allerdings auch der Reaktor so ausgebildet sein, daß die verwendeten Biokatalysatoren sogenannte Suspensionszellen sind, d.h. Zellen, die nur aufgrund von mechanischer Wechselwirkung an der Oberfläche der Microcarrier anhaften.

Bei vorliegendem Ausführungsbeispiel setzt sich die zentrale Umwälzleitung mit einem Fortsatz 5' in den Basisteil 1 fort und ist mit ihrem unteren Ende mit einem sich konisch erweiternden Übergangsteil 9 verbunden. Dieser Übergangsteil verbindet die Umwälzleitung mit einem zylindrischen Kragen 10, in welchem der Rührflügel 11 einer Rühreinrichtung 12 angeordnet ist. Der Rührflügel 11 bildet zusammen mit dem Kragen eineAxialpumpe und ist mittels eines Elektromotors 13, der drehrichtungsumkehrbar ist, in beiden Förderrichtungen antreibbar. Im Inneren des konischen Übergangsteiles 9 sind Schikanen 14 angeordnet, die ein Mitrotieren der Flüssigkeit innerhalb des zylindrischen Kragens 10 verhindern. Im Zwischenraum zwischen dem zylindrischen Kragen 10 und der Wandung des Basisteiles 1 sind gleichfalls Schikanen 15 vorgesehen, mittels welcher die aus dem zylindrischen Kragen unten austretende Flüssigkeit so umgelenkt wird, daß sie vertikal nach oben strömt und damit gleichmäßig durch die poröse Trägerplatte 4 hindurchtritt.

In den Übergangsteil 9 mündet eine Gaseinbringleitung 16 ein, mittels welcher Gas fein verteilt in den zum Rührflügel führenden Flüssigkeitsstrom eingebracht wird. Durch den Rührflügel wird dann das Gas noch mehr zerteilt, und es tritt dann feinst verteilt mit der Flüssigkeit am unteren Ende des zylindrischen Kragens 10 in den Reaktor ein und strömt mit der Flüssigkeit nach oben bis zu der porösen Trägerplatte, wo aufgrund der hydrophoben Ausbildung der Trägeplatte 4 die Gasbläschen, ohne an der Trägesplatte anzuhaften, hindurchtreten können und so das Fließbett bzw. Festbett durchstreifen. Bei diesem Durchstreifen werden aufgrund der Reibung der Gasbläschen an den Füllkörpern 8 jeweils neue Gasaustauschoberflächen geschaffen, wodurch ein gesteigerter Gas/Flüssig-Massen-Transfer erzielt wird. Es wird damit z.B. bis zum oberen Ende des Reaktors immer eine ausreichende Menge an Sauerstoff zur Verfügung stehen. Mit 17 ist ein Anschlußstutzen für Probezieheinrichtungen, Meßelektroden wie Sauerstoffelektrode, pH-Elektrode u.dgl. bezeichnet. Proben können auch bei der Probenentnahmevorrichtung 18 oder aber bei einem Reservestutzen 19, der auch zur Gaseinbringung verwendbar ist, entnommen werden.

Bei 20 sind Schaugläser angedeutet, mit welchen der genaue Verlauf der Strömungsverhältnisse innerhalb des Fließbettes beobachtet werden kann.

Um auch solche Zellen in dem erfindungsgemäßen Reaktor züchten zu können, welche teilweise durch den Flüssigkeitsstrom ausgewaschen und dann wieder durch die Umwälzung in das Bett eingebracht werden, ist der Rührflügel 11 der Rühreinrichtung 12 scherkraftarm ausgebildet, d.h. es sind einerseits keine scharfen Schlagkanten vorgesehen, welche die Zellen beschädigen können, und andererseits ist die Geometrie des Pumpflügels so ausgeführt, daß die Förderleistung maximiert und gleichzeitig turbulente und mechanische Schereffekte minimiert sind.

Nachstehend wird die Funktionsweise des erfindungsgemäßen Reaktors anhand von Verfahrensbeispielen erläutert:

### Verfahrensbeispiel 1 (adhärenter Zelltyp); Kultur im Fließbettreaktor:

Das Verfahrensbeispiel 1 illustriert die Kultur einer adhärenten tierischen Zellinie, einer IgG sezernierenden, rekombinanten CHO-Zelle (Quelle IAM) als typischen Fall einer Zellinie, welche das Wachstum und die Erhaltung der metabolischen Aktivität vorzugsweise verankert an eine Trägermatrix benützt.

Die rekombinanten CHO-Zellen wurden im vorliegenden Verfahrensbeispiel 1 mit DMEM/HAM 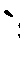 s F12 Medium mit 5% fötalem Kälberserum unter Standardbedingungen (37°C, pH 7,1) in einem 10 Liter Laborfließbettreaktor auf porösen Microcarriern kultiviert.

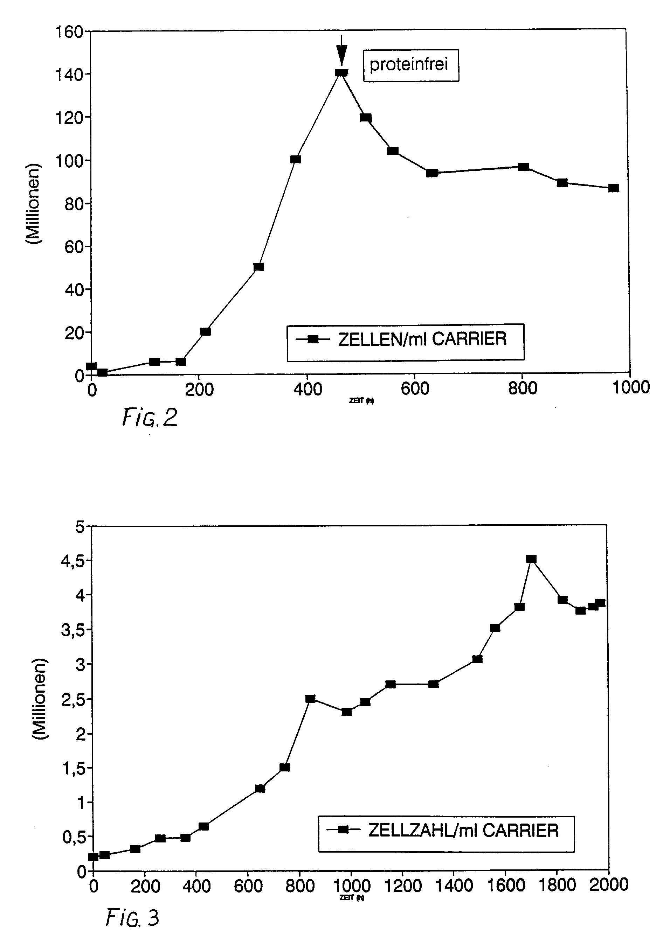

Aus der Figur 2 sind die über einen Zeitraum von 970 Stunden erreichten Zelldichten dargestellt. Die maximal erzielte Zelldichte lag bei 140 x 10⁶ Zellen pro Milliliter poröser Carriermatrix. Der bei Versuchsstunde 466 eingefügte Pfeil weist auf die Verwendung von proteinfreiem Kulturmedium, wodurch lediglich geringe Beeinträchtigung von Wachstum und Metabolismus auftrat.

Dieses Beispiel ist repräsentativ für viele Standardzellinien wie beispielsweise rekombinante und nicht rekombinante CHO, BHK, VERO etc.

### Verfahrensbeispiel 2 (Typus einer Suspensionszelle); Kultur im Fließbettreaktor:

Verfahrensbeispiel 2 zeigt die Kultur einer Suspensionszellinie, einem Maus/Human-Hybrid (Quelle IAM). Sowohl der Immortalisierungspartner wie die mit diesem Immortalisierungspartner fusionierten Zellen sind Zellen des lymphatischen Systems, sie existieren normalerweise als peripher im Blut suspendierte Zellen und dienen im Verfahrensbeispiel 2 als Repräsentanten für "Suspensionszellen".

Die Suspensionszellen wurden im vorliegenden Verfahrensbeispiel 2 mit RPMI 1640 Medium, versetzt mit 2% fötalem Kälberserum, unter Standardbedingungen (37°C, pH 6,95) in einem 10 Liter Labor-Fließbettreaktor auf porösen Microcarriern kultiviert.

Aus der Figur 3 sind die über einen Zeitraum von 2000 Stunden erreichten Zelldichten dargestellt Die erzielte maximale Zelldichte lag bei 4.5 x 10⁶ Zellen pro Milliliter poröser Carriermatrix. Das ist, verglichen mit den in herkömmlichen Reaktortypen (chemostatische Kultur) erreichten Zelldichten eine Erhöhung um den Faktor 10 bis 15.

Das Verfahrensbeispiel 2 läßt den Schluß zu, daß selbst sehr langsam und in niedriger Dichte wachsende Hybridomzellen (Humane Hybride, Xenohybride) mit dem beschriebenen Fließbettreaktorsystem in hohen Zelldichten, d.h. in Zelldichten die wesentlich über 10⁶ Hybriden pro Milliliter liegen, zu kultivieren sind.

### Verfahrensbeispiel 3 (Typus einer Suspensionszelle); Kultur im Festbettreaktor (gepacktes Bett):

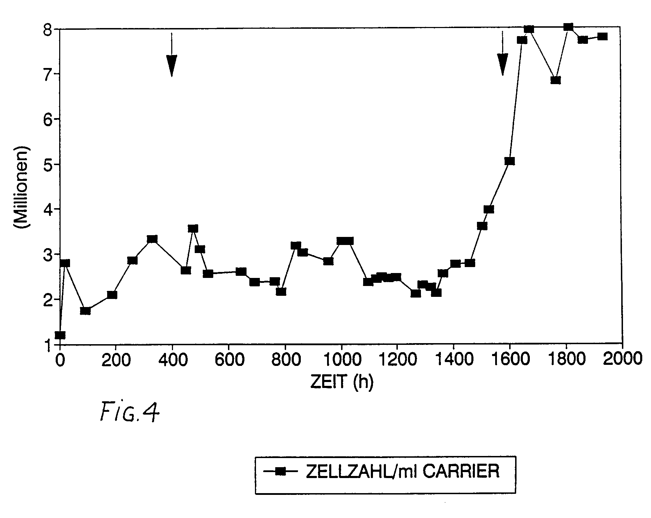

Verfahrensbeispiel 3 zeigt die Kultur einer Suspensionszellinie, (Maus x Maus Hybridom-Zellinie). Die Suspensionszellen wurden im vorliegenden Verfahrensbeispiel 3 mit RPMI 1640 Medium unter Standardbedingungen (37°C, pH 7) in einem 10 Liter Labor-Fließbettreaktor auf porösen Microcarriern kultiviert. Aus der Figur 4 sind die über einen Versuchszeitraum von 2000 Stunden erzielten Zelldichten dargestellt. Die erzielte maximale Zelldichte lag bei 8 x.10⁶ Zellen pro Milliliter poröser Carriermatrix. Die zwischen Versuchsstunde 400 und 1600 angeführten Pfeile weisen auf die Verwendung von serumfreien Perfusionsmedien hin. Aus der Darstellung ist ersichtlich, daß selbst bei Einsatz serumfreier Medien die Kultur über weite Bereiche normales Wachstums und unveränderte metabolische Aktivität aufweist.

Dieses Beispiel läßt den Schluß zu, daß unter Einsatz des beschriebenen Fließbett-bzw. Festbett-Reaktorsystems im Zuge der Kultivierung von Hybridomzellen murinen Ursprungs (Maus x Maus Hybridomzellen) die Erreichung hoher Zelldichten, d.h. Zelldichten die wesentlich über 5 x 10⁶ Zellen pro Milliliter liegen, möglich ist.

## Patentansprüche

1. Reaktor zur Durchführung biologischer Reaktionen mittels Biokatalysatoren mit einem Basisteil, in welchem eine Rühreinrichtung und Überwachungs- und Steuerorgane bzw. -geräte angeordnet sind, und einem dicht aufsetzbaren Deckel, dadurch gekennzeichnet, daß zwischen dem Basisteil (1) und dem Deckel (2) ein Zwischenteil (3) eingesetzt ist, der bei Verwendung als Fließbettreaktor und/oder Festbettreaktor in seinem unteren Bereich einen gasblasendurchlässigen Träger, insbesondere eine Trägerplatte (4), für eine mit Biokatalysatoren beladene Matrix bzw. Biokatalysatormatrix (8) aufweist, wobei eine vom oberen Bereich des Zwischenteiles (3) ausgehende, zum Basisteil führende Umwälzleitung (5) vorgesehen ist, und wobei die Rühreinrichtung (12) als Umwälzpumpe ausgebildet ist.

2. Reaktor nach Anspruch 1 oder 2, dadurch gekennzeichnet, daß der Zwischenteil (3) modular austauschbar ist.

3. Reaktor nach Anspruch 1 oder 2, dadurch gekennzeichnet, daß der durch die Trägerplatte (4) gebildete Träger eine hydrophobe Oberfläche aufweist.

4. Reaktor nach einem der Ansprüche 1 bis 3, dadurch gekennzeichnet, daß der gasblasendurchlässige Träger (4) als statischer Mischer ausgebildet ist.

5. Reaktor nach einem der Ansprüche 1 bis 4, dadurch gekennzeichnet, daß die Umwälzleitung (5) als zentrales, gegebenenfalls durch ein Gitter (7) abgeschlossenes Rohr ausgebildet ist, das die Trägerplatte (4) durchsetzt und zur Rühreinrichtung führt.

6. Reaktor nach Anspruch 5, dadurch gekennzeichnet, daß das die Umwälzleitung bildende zentrale Rohr (5) an seinem unteren Bereich (5') mit einem Kragen (10) versehen ist, der außen das die Pumpe bildende Flügelrad (11) der Rühreinrichtung (12) übergreift, wobei die Rühreinrichtung (12) als Axialförderer ausgebildet ist.

7. Reaktor nach Anspruch 6, dadurch gekennzeichnet, daß zwischen der Umwälzleitung (5') und dem Kragen (10) ein sich konisch zum Kragen (10) hin erweiternder Übergangsbereich (9) vorgesehen ist.

8. Reaktor nach Anspruch 6, dadurch gekennzeichnet, daß der Flügel (11) der Rühreinrichtung (12) etwa in der Längsmitte des Kragens (10) angeordnet ist.

9. Reaktor nach einem der Ansprüche 1 bis 8, dadurch gekennzeichnet, daß in dem Zwischenraum zwischen Kragen (10) und Reaktorinnenwandung Schikanen (14) zur Verhinderung des Rotierens der Reaktorflüssigkeit eingesetzt sind.

10. Reaktor nach einem der Ansprüche 1 bis 9, dadurch gekennzeichnet, daß die Wandung der Umwälzleitung (5) wenigstens in Teilbereichen derselben als Filtermembran, z.B. Ultrafiltrationsmembran, oder als Permeationsmembran ausgebildet ist.

11. Reaktor nach einem der Ansprüche 7 bis 10, dadurch gekennzeichnet, daß in der Umwälzleitung (5) oberhalb der Rühreinrichtung (12), insbesondere im erweiterten Übergangsbereich (9), Gaseinbringorgane (16) vorgesehen sind.

12. Reaktor nach einem der Ansprüche 6 bis 11, dadurch gekennzeichnet, daß das Flügelrad (11) der Rühreinrichtung (12) scherkraftarm ausgebildet ist.

13. Reaktor nach einem der Ansprüche 6 bis 12, dadurch gekennzeichnet, daß die Rühreinrichtung (12) drehrichtungsumkehrbar angetrieben ist.

## Claims

1. Reactor for carrying out biological reactions by means of biocatalysts with a base portion, in which a stirring device and monitoring and control elements or devices are disposed, and a cover which may be placed thereon in a sealed manner, characterised in that inserted between the base portion (1) and the cover (2) there is an intermediate portion (3) which, when used as a fluidised bed reactor and/or solid bed reactor, has in its lower region a carrier, particularly a carrier plate (4), which is permeable to gas bubbles, for a matrix loaded with biocatalysts or a biocatalyst matrix (8), whereby a circulating conduit (5) is provided starting from the upper region of the intermediate portion (3) and leading to the base portion and whereby the stirring device (12) is constructed as a circulating pump.

2. Reactor as claimed in Claim 1 or 2, characterised in that the intermediate portion (3) is replaceable in a modular manner.

3. Reactor as claimed in Claim 1 or 2, characterised in that the carrier constituted by the carrier plate (4) has a hydrophobic surface.

4. Reactor as claimed in one of Claims 1 to 3, characterised in that the carrier (4), which is permeable to gas bubbles, is constructed as a static mixer.

5. Reactor as claimed in one of Claims 1 to 4, characterised in that the circulating conduit (5) is constructed as a central pipe which is optionally closed by a grid (7) and which passes through the carrier plate (4) and leads to the stirring device.

6. Reactor as claimed in Claim 5, characterised in that the central pipe (5) constituting the circulating conduit is provided at its lower region (5') with a collar (10) which extends externally around the impeller (11), constituting the pump, of the stirring device (12), the stirring device (12) being constructed as an axial pump.

7. Reactor as claimed in Claim 6, characterised in that provided between the circulating conduit (5') and the collar (10) there is a transition region (9) which broadens conically towards the collar (10).

8. Reactor as claimed in Claim 6, characterised in that the impeller (11) of the stirring device (12) is arranged approximately on the longitudinal centre of the collar (10).

9. Reactor as claimed in one of Claims 1 to 8, characterised in that inserted into the space between the collar (10) and inner wall of the reactor are baffle plates (14) to prevent rotation of the reactor liquid.

10. Reactor as claimed in one of Claims 1 to 9, characterised in that the wall of the circulating conduit (5) is constructed, at least in portions of the same, as a filter membrane, e.g. an ultrafiltration membrane or as a permeation membrane.

11. Reactor as claimed in one of Claims 7 to 10, characterised in that gas introduction elements (16) are provided in the circulating conduit (5) above the stirring device (12), particularly in the broadened transition region (9).

12. Reactor as claimed in one of Claims 6 to 11, characterised in that the impeller (11) of the stirring device (12) is of low shear force construction.

13. Reactor as claimed in one of Claims 6 to 12, characterised in that the stirring device (12) is driven in a directionally reversible manner.

## Revendications

1. Réacteur servant à la réalisation de réactions biologiques au moyen de biocatalyseurs comportant une partie de base, dans laquelle sont disposés un dispositif agitateur et des organes ou appareils de surveillance et de commande, et un couvercle s'appliquant de façon étanche, caractérisé en ce qu'est insérée, entre la partie de base (1) et le couvercle (2), une partie intermédiaire (3) qui, lorsqu'elle est utilisée comme réacteur à lit fluidifié et/ou réacteur à lit fixe, présente, dans sa zone inférieure, un support perméable aux bulles de gaz, en particulier une plaque de support (4) destinée à une matrice chargée en biocatalyseurs ou matrice de biocatalyseurs (8), une conduite de circulation (5) partant de la zone supérieure de la partie intermédiaire (3) et conduisant à la partie de base étant prévue, le dispositif agitateur (12) étant conformé en pompe de circulation.

2. Réacteur selon la revendication 1 ou 2, caractérisé en ce que la partie intermédiaire (3) est interchangeable de façon modulaire.

3. Réacteur selon la revendication 1 ou 2, caractérisé en ce que le support constitué par la plaque de support (4) présente une surface hydrophobe.

4. Réacteur selon l'une des revendications 1 à 3, caractérisé en ce que le support (4) perméable aux bulles de gaz est conformé en mélangeur statique.

5. Réacteur selon l'une des revendications 1 à 4, caractérisé en ce que la conduite de circulation (5) est conformée en tube central, obturé, le cas échéant, par une grille (7), qui traverse la plaque de support et conduit au dispositif agitateur.

6. Réacteur selon la revendication 5, caractérisé en ce que le tube central (5) constituant la conduite de circulation est pourvu, dans sa zone inférieure (5'), d'un col (10) qui coiffe, par l'extérieur, la roue-hélice (11) formant la pompe du dispositif agitateur (12), le dispositif agitateur (12) étant conformé en transporteur axial.

7. Réacteur selon la revendication 6, caractérisé en ce qu'est prévu, entre la conduite de circulation (5') et le col (10), une zone de transition (9) s'élargissant de façon conique en direction du col (10).

8. Réacteur selon la revendication 6, caractérisé en ce que l'hélice (11) du dispositif agitateur (12) est disposée approximativement au milieu de la longueur du col (10).

9. Réacteur selon l'une des revendications 1 à 8, caractérisé en ce que sont prévues, dans l'interstice entre le col (10) et la paroi intérieure du réacteur, des chicanes (14) destinées à empêcher la rotation du liquide du réacteur.

10. Réacteur selon l'une des revendications 1 à 9, caractérisé en ce que la paroi de la conduite de circulation (5) est conformée, au moins dans certaines parties de celle-ci, en membrane filtrante, par exemple en membrane d'ultrafiltration, ou en membrane de perméation.

11. Réacteur selon l'une des revendications 7 à 10, caractérisé en ce que des organes (16) introduisant le gaz sont prévus dans la conduite de circulation (5), au-dessus du dispositif agitateur (12), en particulier dans la zone de transition (9) élargie.

12. Réacteur selon l'une des revendications 6 à 11, caractérisé en ce que la roue-hélice (11) du dispositif agitateur (12) est dépourvu d'efforts de cisaillement.

13. Réacteur selon l'une des revendications 6 à 12, caractérisé en ce que le dispositif agitateur (12) est entraîné avec possibilité d'inverser le sens de rotation.
